# EUROPEAN PATENT APPLICATION

(11) **EP 4 130 275 A1**
(43) Date of publication of application: **08.02.2023**
(21) Application number: 21779625.9
(22) Date of filing: 31.03.2021
(51) Int. Cl.: C12N 15/55, C12Q 1/6837, C12Q 1/686, C12Q 1/689

(54) **PRIMER SET AND PROBE FOR DETECTING STAPHYLOCOCCUS ARGENTEUS**

(30) Priority: 31.03.2020 JP 2020062646
(71) Applicant: Denka Company Limited, Tokyo 103-8338 (JP); Toho University, Tokyo 143-8540 (JP)
(72) Inventor: TATEDA, Kazuhiro, Ota-ku, Tokyo 143-8541 (JP); MIYATAKE, Yuya, Chuo-ku, Tokyo 103-8338 (JP)
(74) Representative: MacLean, Martin Robert
(86) International application number: PCT/JP2021/013773
(87) International publication number: WO 2021/201072

(57) **Abstract**

The present invention provides a primer set for detecting the presence of *Staphylococcus argenteus* in a specimen, the primer set comprising: a first primer; and a second primer, in which the first primer and the second primer target a nuc gene of *Staphylococcus argenteus.*

## Description

### Technical Field

The present invention relates to a primer set and a probe for detecting the presence of *Staphylococcus argenteus.*

### Background Art

As a method for detecting infectious disease-causing bacteria, a culture identification method in which causative bacteria are isolated and cultured and the bacteria are identified based on their biochemical properties and a genetic method for amplifying and detecting causative bacterium-specific genes through a genetic method such as a PCR method are known, for example.

*Staphylococcus argenteus* is a low frequency bacterial species rarely observed in specimens such as blood. Its clinical significance has not yet been revealed. However, when *Staphylococcus argenteus* is measured through a genetic method such as real-time PCR, it is problematic in that *Staphylococcus argenteus* is likely to be misidentified with other *Staphylococcus* spp. such as *Staphylococcus aureus* which causes food poisoning (Patent Documents 1 and 2).

### Citation List

### Patent Document

Patent Document 1: Patent Publication JP-A-2017-163969
Patent Document 2: Patent Publication JP-A-2017-163970

### Summary

### Technical Problem

With the foregoing in view, it is an object of the present invention to provide a primer set and a probe for detecting the presence of *Staphylococcus argenteus.*

### Solution to Problem

The present inventors have focused on a sequence of a thermostable nuclease (nuc) gene of *Staphylococcus argenteus* and have found a sequence capable of differentiating *Staphylococcus argenteus* which often gave false-negative results in the past from other *Staphylococcus* spp., thus leading to realization of the present invention.

That is, the present application encompasses the following inventions.
[1] A primer set for detecting the presence of *Staphylococcus argenteus* in a specimen, the primer set comprising a first primer and a second primer that target a nuc gene of *Staphylococcus argenteus..*
[2] The primer set according to [1],
   wherein the nuc gene has a base sequence shown in SEQ ID NO: 3 or 4 or a base sequence in which one or several bases are deleted, substituted, inserted, or added in the base sequence shown in any of SEQ ID NOs: 3 and 4, and encodes TNase.
[3] The primer set according to [1] or [2],
   wherein the target nuc gene has a base sequence comprising at least 10 consecutive bases in a base sequence from position 217 to position 331 of the base sequence shown in SEQ ID NO: 3 or 4.
[4] The primer set according to any one of [1] to [3],
   wherein the first primer has a base sequence shown in SEQ ID NO: 9 or a base sequence comprising at least 10 consecutive bases in a base sequence in which one or several bases are deleted, substituted, inserted, or added in the base sequence shown in SEQ ID NO: 9, and has the same function as that of the base sequence shown in SEQ ID NO: 9.
[5] The primer set according to any one of [1] to [4],
   wherein the second primer has a base sequence shown in SEQ ID NO: 10 or a base sequence comprising at least 10 consecutive bases in a base sequence in which one or several bases are deleted, substituted, inserted, or added in the base sequence shown in SEQ ID NO: 10, and has the same function as that of the base sequence shown in SEQ ID NO: 10.
[6] The primer set according to any one of [1] to [5], further comprising: a third primer that targets a nuc gene of *Staphylococcus schweitzeri.*
[7] The primer set according to any one of [1] to [6],
   wherein the third primer has a base sequence shown in SEQ ID NO: 11 or a base sequence comprising at least 10 consecutive bases in a base sequence in which one or several bases are deleted, substituted, inserted, or added in the base sequence shown in SEQ ID NO: 11, and has the same function as that of the base sequence shown in SEQ ID NO: 11.
[8] The primer set according to any one of [1] to [7], further comprising a fourth primer and a fifth primer that target a nuc gene of *Staphylococcus aureus.*
[9] The primer set according to any one of [1] to [8],
   wherein the fourth primer has a base sequence shown in SEQ ID NO: 6 or a base sequence comprising at least 10 consecutive bases in a base sequence in which one or several bases are deleted, substituted, inserted, or added in the base sequence shown in SEQ ID NO: 6, and has the same function as that of the base sequence shown in SEQ ID NO: 6.
[10] The primer set according to any one of [1] to [9],
   wherein the fifth primer has a base sequence shown in SEQ ID NO: 7 or a base sequence comprising at least 10 consecutive bases in a base sequence in which one or several bases are deleted, substituted, inserted, or added in the base sequence shown in SEQ ID NO: 7, and has the same function as that of the base sequence shown in SEQ ID NO: 7.
[11] The primer set according to any one of [1] to [10],
   wherein the primers are labeled with biotin.
[12] A probe for detecting the presence of *Staphylococcus argenteus* in a specimen, in which the probe targets a nuc gene of *Staphylococcus argenteus.*
[13] The probe according to [12],
   wherein the nuc gene has a base sequence shown in SEQ ID NO: 3 or 4 or a base sequence in which one or several bases are deleted, substituted, inserted, or added in the base sequence shown in SEQ ID NO: 3 or 4, and encodes Nuc protein.
[14] The probe according to [12] or [13],
   wherein the target nuc gene has a base sequence comprising at least 8 consecutive bases in a base sequence from position 217 to position 331 of the base sequence shown in SEQ ID NO: 3 or 4.
[15] The probe according to any one of [12] to [14],
   wherein the probe has a base sequence shown in SEQ ID NO: 12 or a base sequence comprising at least 10 consecutive bases in a base sequence in which one or several bases are deleted, substituted, inserted, or added in the base sequence shown in SEQ ID NO: 12, and has the same function as that of the base sequence shown in SEQ ID NO: 12.
[16] A kit comprising the primer set according to any one of [1] to [11], and the probe according to any one of [12] to [15].
[17] The kit according to [16], further comprising: a probe targeting the nuc gene of *Staphylococcus aureus.*
[18] The kit according to [17],
   wherein the probe targeting the nuc gene of *Staphylococcus aureus* has a base sequence shown in SEQ ID NO: 8 or a base sequence comprising at least 8 consecutive bases in a base sequence in which one or several bases are deleted, substituted, inserted, or added in the base sequence shown in SEQ ID NO: 8, and has the same function as that of the base sequence shown in SEQ ID NO: 8.
[19] A method for detecting the presence of *Staphylococcus argenteus* in a specimen, the method comprising a step of: using the primer set according to any one of [1] to [11].
[20] The method according to [19], further comprising a step of: differentiating *Staphylococcus argenteus* from *Staphylococcus schweitzeri* or other *Staphylococcus* bacteria using the first, second, and third primers.
[21] The method according to [19] or [20],
   wherein the probe according to any one of [12] to [15] is additionally used.
[22] The method according to any one of [19] to [21],
   wherein the other *Staphylococcus* bacteria include *Staphylococcus aureus* or coagulase-negative *Staphylococcus* bacteria.
[23] The method according to any one of [19] to [22],
   wherein the coagulase-negative *Staphylococcus* bacteria are one or more selected from the group consisting of *Staphylococcus epidermidis, Staphylococcus auricularis, Staphylococcus camosus, Staphylococcus condiment!, Staphylococcus debuckii, Staphylococcus massiliensis, Staphylococcus piscifermentans, Staphylococcus simulans, Staphylococcus capitis, Staphylococcus caprae, Staphylococcus saccharolyticus, Staphylococcus haemolyticus, Staphylococcus devriesei, Staphylococcus hominis, Staphylococcus hyicus, Staphylococcus agnetis, Staphylococcus chromogenes, Staphylococcus cornubiensis*, *Staphylococcus felis, Staphylococcus delphini, Staphylococcus intermedius, Staphylococcus lutrae, Staphylococcus microti, Staphylococcus muscae, Staphylococcus pseudintermedius, Staphylococcus rostri, Staphylococcus schleiferi, Staphylococcus lugudnensis, Staphylococcus saprophyticus, Staphylococcus arlettae, Staphylococcus caeli, Staphylococcus cohnii, Staphylococcus equorum, Staphylococcus gallinarum, Staphylococcus kloosii, Staphylococcus leei, Staphylococcus nepalensis, Staphylococcus succinus, Staphylococcus xylosus, Staphylococcus sciuri, Staphylococcus fleurettii, Staphylococcus lentus, Staphylococcus stepanovicii, Staphylococcus vitulinus, Staphylococcus simulans, Staphylococcus pasteuri,* and *Staphylococcus warned.*
[24] The method according to [23],
   wherein the other *Staphylococcus* bacteria include *Staphylococcus aureus,* and the fourth primer and the fifth primer and/or the probe that target the nuc gene of *Staphylococcus aureus* are additionally used.

### Advantageous Effects of Invention

According to the primers and/or the probe of the present invention, it has become possible to differentiate *Staphylococcus argenteus* which is likely to be misidentified from other *Staphylococcus* bacteria with higher accuracy than before.

### Brief Description of Drawings

Fig. 1 shows multiple alignment results of nuc genes of an NCTC13626 strain and an AR221 strain of S. *aureus* (SEQ ID NOs: 1 and 2), nuc genes of an NCTC13711 strain and a XN0106 strain of S. *argenteus* (SEQ ID NOs: 3 and 4), and a nuc gene of an S. *schweitzeri* FSCB5 strain (SEQ ID NO: 5).
Fig. 2 shows results of proliferation curves of amplification products obtained using a primer set of SEQ ID NOs: 6 and 7. The solid line shows an S. *aureus* JCM5676 strain, and the broken line shows an S. *argenteus* JCM31982 strain.
Fig. 3 shows results of proliferation curves of amplification products obtained using a primer set of SEQ ID NOs: 9 to 11. The solid line shows an S. *aureus* JCM5676 strain, and the broken line shows an S. *argenteus* JCM31982 strain.
Fig. 4 shows on-substrate detection results for genomic DNAs of S. *aureus* and S. *argenteus* using DNA probes. The vertical axis shows fluorescence intensity measured in πCode.
Fig. 5 shows on-substrate detection results for clinical specimens using DNA probes. The vertical axis shows fluorescence intensity measured in rrCode.
Fig. 6 shows results of proliferation curves of amplification products obtained using a primer set of SEQ ID NOs: 6, 7, 9, 10, and 11.
Fig. 7 shows results of proliferation curves of amplification products obtained using a primer set of SEQ ID NOs: 13 and 14.
Fig. 8 shows thermal melting curve analysis results of PCR products when the primer set of SEQ ID NOs: 6, 7, 9, 10, and 11 is used.
Fig. 9 shows thermal melting curve analysis results of PCR products when the primer set of SEQ ID NOs: 13 and 14 is used.
Fig. 10 shows on-substrate detection results for genomic DNAs of S. *aureus* and S. *argenteus* using the primer set of SEQ ID NOs: 6, 7, 9, 10, and 11 and the primer set of SEQ ID NOs: 13 and 14. The vertical axis shows fluorescence intensity measured in πCode.

### Description of Embodiments

Hereinafter, the embodiment of the present invention (hereinafter referred to as the "present embodiment") will be described, but the scope of the present invention should not be limitedly interpreted by the embodiment below.

### (Primer Set)

In a first aspect, there is provided a primer set for detecting the presence of *Staphylococcus argenteus* in a specimen. The primer set comprises a first primer and a second primer which target a nuc gene of *Staphylococcus argenteus.*

A nuc gene in *Staphylococcus* bacteria encodes TNase which is a thermostable nuclease specific to *Staphylococcus aureus* in the genus *Staphylococcus,* and is known to be suitable as a target gene in genetic methods (Detection of Staphylococcus aureus by polymerase chain reaction amplification of the nuc gene. J. Clin. Microbiol. 30:1654-1660, 1992).

**Specimens** are not particularly limited as long as the presence of *Staphylococcus* spp. including *Staphylococcus argenteus* is suspected, and examples thereof include body fluids such as human or non-human blood, serum, plasma, urine, feces, saliva, sputum, interstitial fluid, cerebrospinal fluid, and swabs and dilutions thereof, and blood, serum, plasma, urine, feces, cerebrospinal fluids or dilutions thereof are preferable. The specimen may be foodstuffs, soil, plants or the like.

Examples of nuc genes of *Staphylococcus argenteus* include a nuc gene which has a base sequence shown in SEQ ID NO: 3 or 4 or a base sequence in which one or several bases are deleted, substituted, inserted, or added in the base sequence shown in SEQ ID NO: 3 or 4, and encodes TNase.

For example, the first primer as a forward primer may have any sequence as long as it can hybridize with a base sequence shown in SEQ ID NO: 3 or 4 or a base sequence in which one or several bases are deleted, substituted, inserted, or added in the base sequence shown in SEQ ID NO: 3 or 4, under stringent conditions.

Similarly, the second primer as a reverse primer may have any sequence as long as it can hybridize with a complementary strand of a base sequence shown in SEQ ID NO: 3 or 4 or a base sequence in which one or several bases are deleted, substituted, inserted, or added in the complementary strand of the base sequence shown in SEQ ID NO: 3 or 4, under stringent conditions.

**As** used herein, "stringent conditions" means conditions in which a so-called specific hybrid is formed and a non-specific hybrid is not formed, and for example, can be appropriately determined based on information such as the length of the base sequence. For example, stringent conditions can be set with reference to Molecular Cloning (Fourth Edition, Cold Spring Harbor Laboratory Press, New York). The stringent conditions can be called conditions in which polynucleotides having a high homology (preferably, identity), for example, polynucleotides having a homology of 70% or more, preferably 80% or more, more preferably 90% or more, still more preferably 95% or more, and particularly preferably 99% or more are hybridized, and polynucleotides having a lower homology are not hybridized.

Specific stringent conditions are conditions including incubating a specimen having a base sequence complementary to a desired base sequence and suspected of having *Staphylococcus argenteus* in a hybridization solution (50% formamide, 10×SSC (0.15 M NaCl, 15 mM sodium citrate, pH 7.0), a 5× Denhardt's solution, 1% SDS, 10% dextran sulfate, 10 µg/ml of denatured Salmon Sperm DNA, and a 50 mM phosphate buffer (pH 7.5)) at about 42°C to about 50°C, and then washing using 0.1×SSC, 0.1% SDS at about 65°C to about 70°C.

Primers and probes are designed so that they do not cause a cross reaction with sequences of other related species, but can be appropriately changed according to the system used for detecting an amplification product and the like, and other conditions. When an amplification product having a length of 100 to 200 bases is generated, for example, it is preferable to target a base sequence from position 217 to position 331 of the base sequence shown in SEQ ID NO: 3 or 4. Primers and probes are designed to have a base sequence comprising at least 10 consecutive bases in the base sequence constituting such a target. It is preferable to design primers so that there is a mismatch with a *Staphylococcus* bacterium other than *Staphylococcus argenteus* at the end of the primer, particularly at the 3' end.

Examples of first primers targeting a nuc gene of *Staphylococcus argenteus* include one which has a base sequence shown in SEQ ID NO: 9 or a base sequence comprising at least 10 consecutive bases in a base sequence in which one or several bases are deleted, substituted, inserted, or added in the base sequence shown in SEQ ID NO: 9, and has the same function as that of the base sequence shown in SEQ ID NO: 9.
5'-GGTGAACACCCTGGTCTC-3' (SEQ ID NO: 9)

Examples of second primers targeting a nuc gene of *Staphylococcus argenteus* include one which has a base sequence shown in SEQ ID NO: 10 or a base sequence comprising at least 10 consecutive bases in a base sequence in which one or several bases are deleted, substituted, inserted, or added in the base sequence shown in SEQ ID NO: 10, and has the same function as that of the base sequence shown in SEQ ID NO: 10.
5'-GATATTCCGGCCCCATAATG-3' (SEQ ID NO: 10)

**The** length of the first and second primers is arbitrary, and can be, for example, appropriately set to be within a range of about 10 bases to about 35 bases, and is preferably at least 10 bases or more.

**The** ratio of the first primer and the second primer is not particularly limited, and may be the same as or different from each other. When the primer set is used in an asymmetric PCR method, the concentration of one primer can be adjusted to be higher than the concentration of the other primer.

*Staphylococcus* bacteria are classified into coagulase-positive ones and coagulase-negative ones. For example, *Staphylococcus argenteus* is coagulase positive. Coagulase-negative *Staphylococcus* bacteria (CNS) include a *Staphylococcus* bacterium called *Staphylococcus epidermidis* frequently found in clinical materials, and it is necessary for *Staphylococcus argenteus* to be differentiated from such coagulase-negative *Staphylococcus* bacteria as well. Since coagulase-negative *Staphylococcus* bacteria do not have a nuc gene fragment, these are suitable to be differentiated.

Primers based on a base sequence of SEQ ID NO: 10 can also be used as forward primers for detecting the presence of *Staphylococcus schweitzeri.* Coagulase-positive *Staphylococcus schweitzeri* is related to *Staphylococcus aureus,* but is known to be a species closer to *Staphylococcus argenteus* in consideration of its sequence.

The first and second primers can specifically amplify all or a part of a nuc gene of *Staphylococcus argenteus.* Accordingly, *Staphylococcus argenteus* can be differentiated from other *Staphylococcus* spp. Alternatively, amplification products of the first and second primers can be subjected to melting curve analysis, and the melting temperatures thereof can be compared to those of amplification products of other primer sets to differentiate *Staphylococcus argenteus* from other *Staphylococcus* spp.

The first and second primers can specifically amplify a part of a nuc gene of *Staphylococcus argenteus.* Accordingly, *Staphylococcus argenteus* can be differentiated from other *Staphylococcus* spp. The other *Staphylococcus* spp. include *Staphylococcus aureus* or coagulase-negative *Staphylococcus* bacteria.

The genus *Staphylococcus* is currently classified into 36 species and 19 subspecies, and is widely distributed in birds and mammals including livestock and humans in nature. *Staphylococcus aureus* is the most pathogenic staphylococcal genus and causes staphylococcal food poisoning or suppurative diseases in humans. *Staphylococcus aureus* is coagulase positive.

The coagulase-negative *Staphylococcus* bacteria are not limited, but include *Staphylococcus auricularis, Staphylococcus carnosus, Staphylococcus condiment!, Staphylococcus debuckii, Staphylococcus massiliensis, Staphylococcus piscifermentans, Staphylococcus simulans, Staphylococcus epidermidis, Staphylococcus capitis, Staphylococcus caprae, Staphylococcus saccharolyticus, Staphylococcus haemolyticus, Staphylococcus devriesei, Staphylococcus hominis, Staphylococcus hyicus, Staphylococcus agnetis, Staphylococcus chromogenes, Staphylococcus cornubiensis, Staphylococcus felis, Staphylococcus delphini, Staphylococcus intermedius, Staphylococcus lutrae, Staphylococcus microti, Staphylococcus muscae, Staphylococcus pseudintermedius, Staphylococcus rostri, Staphylococcus schleiferi, Staphylococcus lugudnensis, Staphylococcus saprophyticus, Staphylococcus arlettae, Staphylococcus caeli, Staphylococcus cohnii, Staphylococcus equorum, Staphylococcus gallinarum, Staphylococcus kloosii, Staphylococcus leei, Staphylococcus nepalensis, Staphylococcus succinus, Staphylococcus xylosus, Staphylococcus sciuri*, *Staphylococcus fleurettii, Staphylococcus lentus, Staphylococcus stepanovicii, Staphylococcus vitulinus, Staphylococcus simulans, Staphylococcus pasteuri,* and *Staphylococcus warneri.*

A primer set may contain additional primers, for example, a third primer, a fourth primer, a fifth primer, and a sixth primer, in addition to the first and second primers. Detection sensitivity can be improved by using appropriate additional primers. Examples of such primers include primers for detecting *Staphylococcus argenteus* or other *Staphylococcus* spp. expected to be contained in a specimen. Examples of other *Staphylococcus* spp. include *Staphylococcus schweitzeri.*

Examples of forward primers for detecting *Staphylococcus schweitzeri* include those described above for SEQ ID NO: 10, and examples of reverse primers include one which has a base sequence shown in SEQ ID NO: 11 or a base sequence comprising at least 10 consecutive bases in a base sequence in which one or several bases are deleted, substituted, inserted, or added in the base sequence shown in SEQ ID NO: 11, and has the same function as that of the base sequence shown in SEQ ID NO: 11.

The presence of *Staphylococcus argenteus* contained in a specimen can be detected through a nucleic acid amplification reaction using the above-described primers, and *Staphylococcus argenteus* can be differentiated from other *Staphylococcus* bacteria. For example, the accuracy of the discrimination can be improved using a fourth primer or a fifth primer targeting a nuc gene of *Staphylococcus aureus.*

Examples of fourth primers include one which has a base sequence shown in SEQ ID NO: 6 or a base sequence comprising at least 10 consecutive bases in a base sequence in which one or several bases are deleted, substituted, inserted, or added in the base sequence shown in SEQ ID NO: 6, and has the same function as that of the base sequence shown in SEQ ID NO: 6.

Examples of fifth primers include one which has a base sequence shown in SEQ ID NO: 7 or a base sequence comprising at least 10 consecutive bases in a base sequence in which one or several bases are deleted, substituted, inserted, or added in the base sequence shown in SEQ ID NO: 7, and has the same function as that of the base sequence shown in SEQ ID NO: 7.

Nucleic acid amplification methods include a PCR method, and examples thereof include a multiplex PCR, a loop-mediated isothermal amplification (LAMP) method, an isothermal and chimeric primer-initiated amplification of nucleic acids (ICAN) method, a rolling circle amplification (RCA) method, a ligase chain reaction (LCR) method, and a strand displacement amplification (SDA) method. When it is expected that a large number of *Staphylococcus* bacteria are contained in the specimen, a multiplex PCR is preferable because a plurality of bacteria can be comprehensively and simultaneously detected.

Regarding a method of detecting an amplification product, known methods such as an electrophoresis method using polyacrylamide or agarose gel can be performed. For example, in electrophoresis, the presence of an amplification product can be identified based on the mobility of the amplification product with respect to the mobility of a marker having a known molecular weight.

A label that can specifically recognize an amplification product may be used for detection of the amplification product after the nucleic acid amplification reaction. Examples of such labels include a fluorescent dye, biotin, and digoxigenin. When fluorescence is used as the label, the fluorescence can be detected using a fluorescence microscope, a fluorescence plate reader or the like.

### (Probe)

In a second aspect, there is provided a probe for detecting the presence of *Staphylococcus argenteus* in a specimen. The probe can target a nuc gene of *Staphylococcus argenteus.*

The probe is preferably designed such that the interior of the probe includes a mismatch with a *Staphylococcus* bacterium other than *Staphylococcus argenteus.* Thereby, the base length of the full-match part during hybridization can be shortened, the specificity can be improved, and false positives can be reduced.

Examples of probes for *Staphylococcus argenteus* or *Staphylococcus schweitzeri* include one which has a base sequence shown in SEQ ID NO: 12 or a base sequence comprising at least 8 consecutive bases in a base sequence in which one or several bases are deleted, substituted, inserted, or added in the base sequence shown in SEQ ID NO: 12, and has the same function as that of the base sequence shown in SEQ ID NO: 12.
5'-CATATTTTTCAACGCCT-3' (SEQ ID NO: 12)

Probes for *Staphylococcus* bacteria other than *Staphylococcus argenteus* or *Staphylococcus schweitzeri* may be combined therewith. Examples of probes for *Staphylococcus aureus* include one which has a base sequence shown in SEQ ID NO: 8 or a base sequence comprising at least 50 consecutive bases in a base sequence in which one or several bases are deleted, substituted, inserted, or added in the base sequence shown in SEQ ID NO: 18, and has the same function as that of the base sequence shown in SEQ ID NO: 8.

The probe may be bound to a solid-phase component such as beads. Examples of such a solid-phase component include beads suitable for multiplex assays, for example, beads having a unique analog code identifier for storing information for multiplex assays as disclosed in WO2018052464A1.

### (Kit)

In a third aspect, there is provided a kit comprising a primer set for detecting the presence of *Staphylococcus argenteus* in a specimen and a probe for detecting the presence of *Staphylococcus argenteus* in a specimen.

The kit may comprise a reagent used for detecting *Staphylococcus argenteus,* for example, a reagent for performing an amplification reaction. The reagent for performing an amplification reaction contains, for example, a buffer solution, salts, primers, deoxyribonucleotides, and thermostable DNA polymerase. The content and reagent of the kit can be appropriately determined by those skilled in the art according to the amplification method, and the kit may further comprise a solid-phase component for hybridization with the amplification product obtained using the primer or the probe.

The kit may further contain primers or probes for detecting *Staphylococcus aureus, Staphylococcus epidermidis,* or other *Staphylococcus* spp. expected to be contained in a specimen. Examples of primers and probes for detecting *Staphylococcus aureus* include primers having base sequences shown in SEQ ID NOs: 6 and 7 and a probe having a base sequence shown in SEQ ID NO: 8.

### (Detection Method)

In a fourth aspect, there is provided a method for detecting the presence of *Staphylococcus argenteus* in a specimen. The above-described primer set and/or probes can be used for detecting the presence of *Staphylococcus argenteus.*

*Staphylococcus argenteus* can be differentiated from *Staphylococcus aureus* or other *Staphylococcus* bacteria using a primer set of SEQ ID NOs: 9 and 10. Presence of *Staphylococcus schweitzeri* can also be detected by combining a forward primer of SEQ ID NO: 11 with a reverse primer of SEQ ID NO: 10.

The specimen may be further subjected to average nucleotide identity (ANI) analysis to determine the difference between detected bacteria.

The present invention will be described below in detail with reference to examples, but the present invention is not limited thereto.

### [Examples]

### Example 1: On-Substrate Detection for Genomic DNAs of S. Aureus and S. Argenteus Using DNA Probes

### Design of Primers and Probes

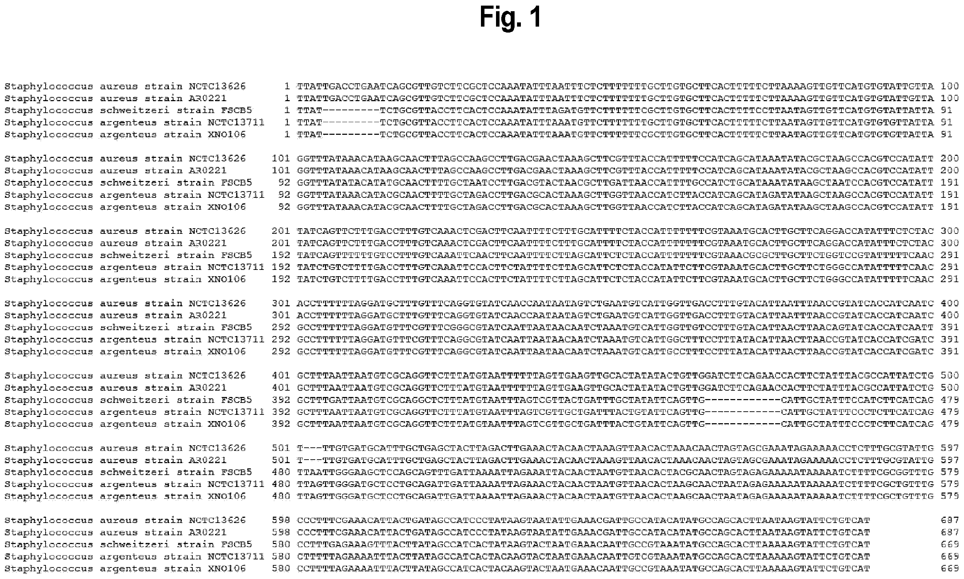

Primers and probes of SEQ ID NOs: 6 to 12 were designed from multiple alignment results of nuc genes of an NCTC13626 strain and an AR221 strain of S. *aureus* (SEQ ID NOs: 1 and 2), nuc genes of an NCTC13711 strain and a XN0106 strain of S. *argenteus* (SEQ ID NOs: 3 and 4), and a nuc gene of an S. *schweitzeri* FSCB5 strain (SEQ ID NO: 5) shown in Fig. 1.

**[Table 1]**

| | |
|---|---|
| SEQ ID NO: 6: *S*. *aureus* primer | GGTGAACACCCTGGTTTC |
| SEQ ID NO: 7: *S*. *aureus* primer | CGATATTCCGGCCCCATG |
| SEQ ID NO: 8: *S*. *aureus* probe | GTTGCAGCAGTTTGAG |
| SEQ ID NO: 9: *S*. *argenteus* primer | GGTGAACACCCTGGTCTC |
| SEQ ID NO: 10: *S*. *argenteus*/*schweitzeri* primer | GATATTCCGGCCCCATAATG |
| SEQ ID NO: 11: *S*. *schweitzeri* primer | GAACTGCAGCATTTTG |
| SEQ ID NO: 12: *S*. *argenteus*/*schweitzeri* probe | GAACTGCAGCATTTTG |

### Confirmation of Nucleic Acid Amplification

Genomic DNAs extracted from an S. *aureus* JCM5676 strain and an S. *argenteus* JCM31982 strain were used as templates, and amplification by real-time PCR was performed using primers of SEQ ID NOs: 6 and 7 or primers of 9 to 11. A TaKaRa Multiplex Assay Kit Ver. 2 (Takara Bio Inc.) was used as a reagent for amplification, and EvaGreen (Biotium) was used as an intercalator dye. The primers and the genomic DNAs were added so that each primer has a final concentration of 0.2 µM and the genomic DNAs as templates have a concentration of 10 µg/L, a 2× reaction buffer was used so as to become 1× in the TaKaRa Multiplex Assay Kit Ver. 2, 1 U of Multiplex Enzyme Mix was used, and the template DNAs and the like were mixed with each other to prepare a PCR reaction solution. Light Cycler 480 System II (Roche Diagnostics) was used as a measurement device.

The amplification reaction conditions used are as follows.
94°C: 30 seconds
60°C: 60 seconds -> 94°C: 30 seconds (45 cycles)
72°C: 600 seconds

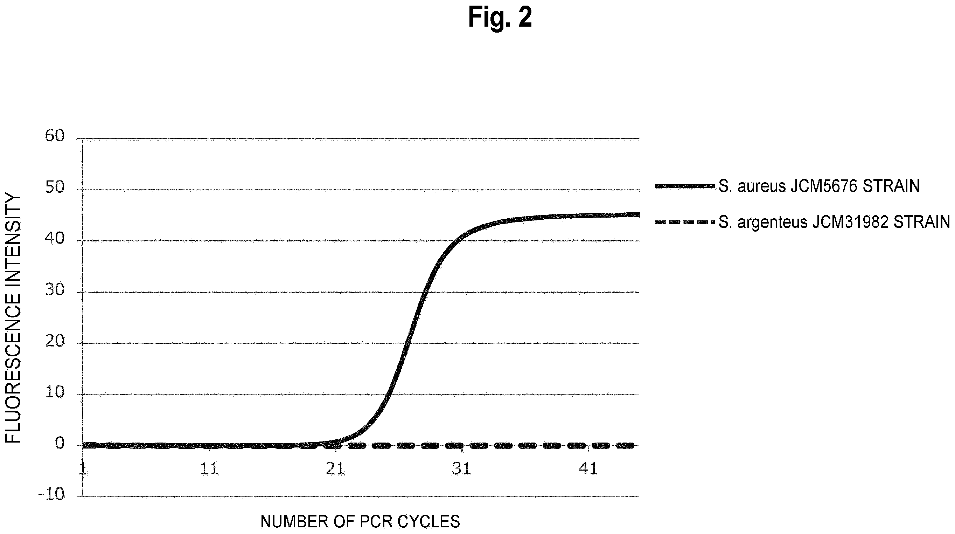

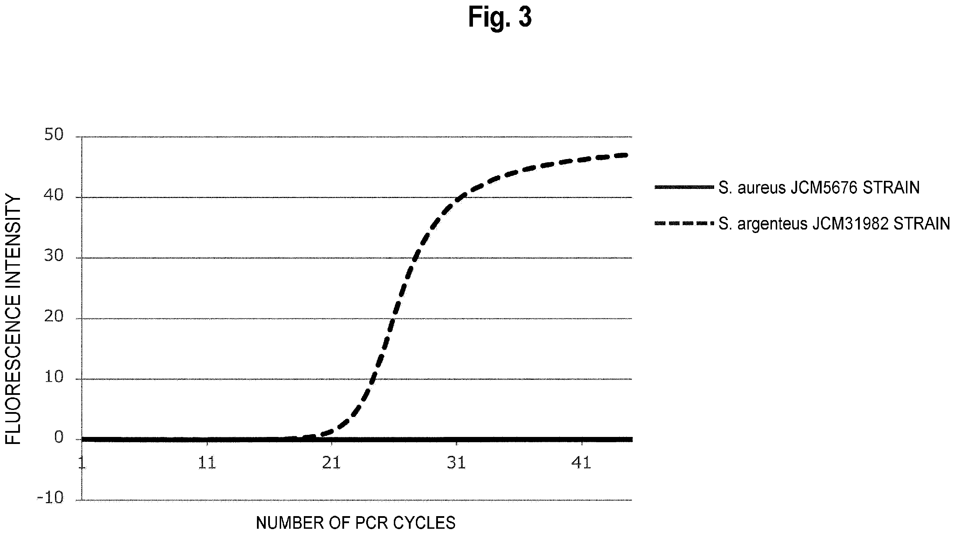

The measurement results when the primers of SEQ ID NOs: 6 and 7 are used are shown in Fig. 2, and the measurement results when the primers of SEQ ID NOs: 9 to 11 are used are shown in Fig. 3. Amplification was confirmed only in the S. *aureus* JCM5676 strain in the primer set of SEQ ID NOs: 6 and 7, whereas amplification was confirmed only in the S. *argenteus* JCM31982 strain in the primer set of SEQ ID NOs: 9 to 11. From the above, it was found that the primer set of SEQ ID NOs: 6 and 7 and the primer set of 9 to 11 have a function of amplifying mutually targeted nucleic acid components.

### Nucleic Acid Amplification and Detection

The amplification by PCR using the S. *aureus* JCM5676 strain and the S. *argenteus* JCM31982 strain and using the primer set of SEQ ID NOs: 6, 7, 9, 10, and 11, and a hybridization reaction, labeling, and fluorescence measurement on substrates were performed. At this time, primers in which the 5' ends of SEQ ID NOs: 7 and 11 were modified with biotin via linkers were used.

A TaKaRa Multiplex Assay Kit Ver. 2 (Takara Bio Inc.) was used for the amplification. The primers and the genomic DNAs were added so that each primer has a final concentration of 0.2 µM and the genomic DNAs as templates have a concentration of 10 µg/L, a 2× reaction buffer was used so as to become 1 × in the TaKaRa Multiplex Assay Kit Ver. 2, 1 U of Multiplex Enzyme Mix was used, and the template DNAs and the like were mixed with each other to prepare a PCR reaction solution. T100 Thermal Cycler (Bio-Rad Laboratories, Inc.) was used as a nucleic acid amplifier. After the completion of the amplification reaction, a thermal denaturation reaction was performed in the same device before the reactants were subjected to a hybridization reaction on substrates.

As a reagent used for the hybridization reaction on substrates, a solution comprising a substrate 1 on which a probe of SEQ ID NO: 8 was immobilized and a substrate 2 on which a probe of SEQ ID NO: 12 was immobilized was mixed with Saline-Sodium Phosphate-EDTA Buffer (SSPE buffer) and the PCR reaction solution which had been subjected to thermal denaturation.

Streptavidin-Phycoerythrin (PlexBio Co., Ltd.) was used as a reagent for labeling. IntelliPlex 1000 πCode Processor (PlexBio Co., Ltd.) was used as a device for the labeling and the hybridization reaction on substrates.

PlexBio (registered trademark) 100 Fluorescent Analayzer (PlexBio Co., Ltd.) was used as a fluorescence measurement device.

The reaction conditions used are as follows.
Amplification reaction conditions
94°C: 30 seconds
60°C: 60 seconds -> 94°C: 30 seconds (30 cycles)
72°C: 600 seconds

Thermal denaturation conditions
95°C: 5 minutes -> rapid cooling to 4°C

Hybridization conditions
37°C: incubation for 20 minutes

Labeling conditions
Incubation at 37°C for 10 minutes

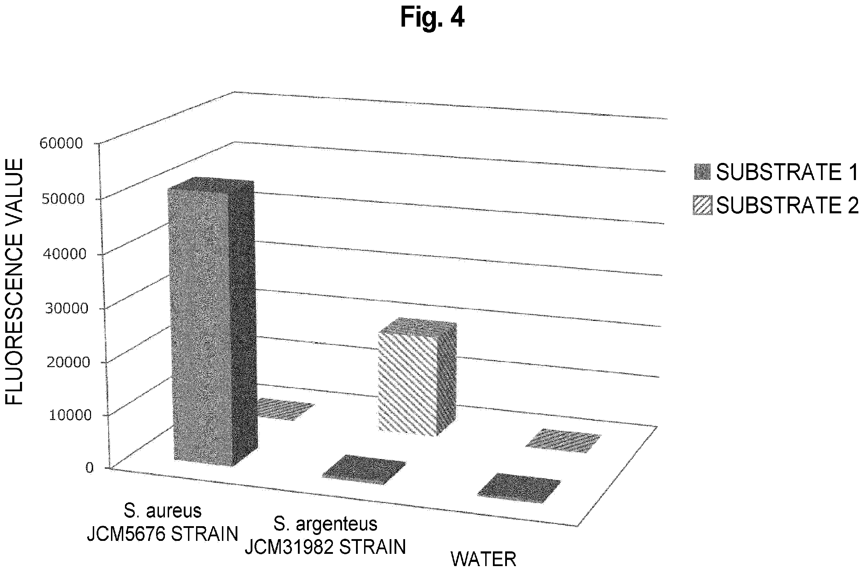

The measurement results are shown in Fig. 4. Fluorescence was observed in the substrate 1 in a case where an S. *aureus* JCM5676 strain was used as a template, and fluorescence was observed in the substrate 2 in a case where an S. *argenteus* JCM31982 strain was used as a template. In addition, in a case where water was used as a template as a negative control, no fluorescence was observed. From the above, it could be expected that the use of the primers of SEQ ID NOs: 6, 7, 9, 10, and 11 and the probes of SEQ ID NOs: 8 and 12 enabled differentiating S. *aureus* from S. *argenteus* which was impossible in the past. In addition, it was possible to confirm that the primer set of SEQ ID NOs: 6, 7, 9, 10, and 11 functioned even when coexisting in the same system.

### Example 2: Detection on Substrates Using DNA Probes for Three Clinical Specimens (Blood Culture Solutions)

### Nucleic Acid Amplification and Detection

Amplification by PCR using a primer set of SEQ ID NOs: 6, 7, 9, 10, and 11 and using genomic DNAs, which were extracted from specimens 1 and 2 identified as S. *aureus* and a specimen 3 identified as S. *epidermidis* through a culture identification method using a QIAamp BiOstic Bacteremia DNA Kit (QIAGEN) according to the standard protocol included in the reagent, as templates, and a hybridization reaction, labeling, and fluorescence measurement on substrates were performed. At this time, primers in which the 5' ends of SEQ ID NOs: 7 and 11 were modified with biotin via linkers were used.

A TaKaRa Multiplex Assay Kit Ver. 2 (Takara Bio Inc.) was used for the amplification. The primers and the genomic DNAs were added so that each primer has a final concentration of 0.2 µM and the genomic DNAs as templates have a concentration of 10 µg/L, a 2× reaction buffer was used so as to become 1× in the TaKaRa Multiplex Assay Kit Ver. 2, 1 U of Multiplex Enzyme Mix was used, and the template DNAs and the like were mixed with each other to prepare a PCR reaction solution. T100 Thermal Cycler (Bio-Rad Laboratories, Inc.) was used as a nucleic acid amplifier. After the completion of the amplification reaction, a thermal denaturation reaction was performed in the same device before the reactants were subjected to a hybridization reaction on substrates.

As a reagent used for the hybridization reaction on substrates, a solution comprising a substrate 1 on which a probe of SEQ ID NO: 8 was immobilized and a substrate 2 on which a probe of SEQ ID NO: 12 was immobilized was mixed with Saline-Sodium Phosphate-EDTA Buffer (SSPE buffer) and the PCR reaction solution which had been subjected to thermal denaturation.

Streptavidin-Phycoerythrin (PlexBio Co., Ltd.) was used as a reagent for labeling. IntelliPlex 1000 rrCode Processor (PlexBio Co., Ltd.) was used as a device for the labeling and the hybridization reaction on substrates.

PlexBio (registered trademark) 100 Fluorescent Analayzer (PlexBio Co., Ltd.) was used as a fluorescence measurement device.

The reaction conditions used are as follows.
Amplification reaction conditions
94°C: 30 seconds
60°C: 60 seconds -> 94°C: 30 seconds (30 cycles)
72°C: 600 seconds

Thermal denaturation conditions
95°C: 5 minutes -> rapid cooling to 4°C

Hybridization conditions
37°C: incubation for 20 minutes

Labeling conditions
37°C: incubation for 10 minutes

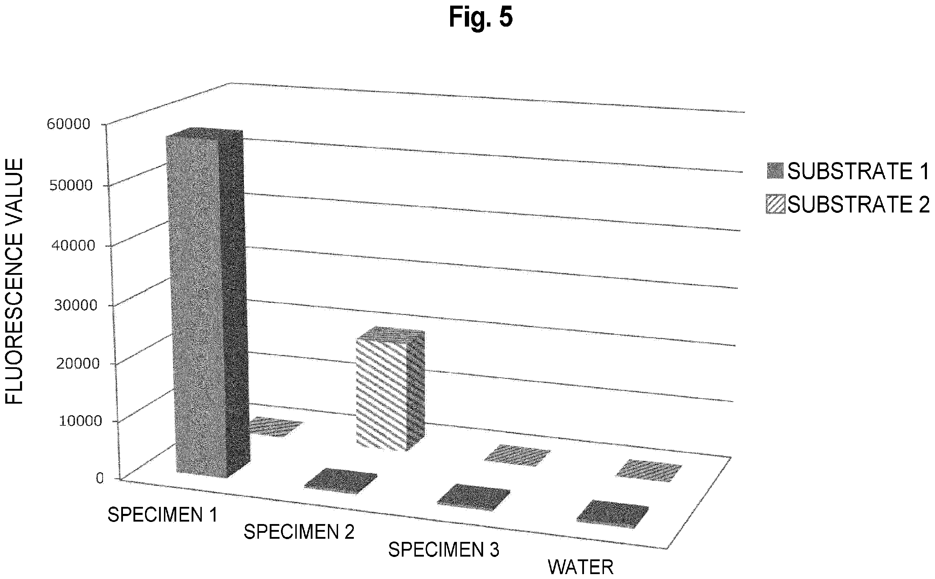

**The** measurement results are shown in Fig. 5. Fluorescence was observed in the substrate 1 in a case where the specimen 1 was used as a template, and fluorescence was observed in the substrate 2 in a case where the specimen 2 was used as a template. From this, it was suggested that there is a possibility that the specimen 2 may be S. *argenteus* in view of Example 1. In Verification 1 below, whole-genome analysis using a next generation sequencer and identification of bacterial species through ANI were performed on the specimens 1 and 2. As a result, it became clear that the specimen 1 was classified as S. *aureus,* whereas the specimen 2 was classified as S. *argenteus.* In combination with Example 1, the use of the primer set of SEQ ID NOs: 6, 7, 9, 10, and 11 and the probes of SEQ ID NOs: 8 and 12 enabled differentiating S. *aureus* from S. *argenteus* which was impossible in the past.

In addition, since fluorescence was not observed from either substrate of the specimen 3, it was found that S. *epidermidis* which was another *Staphylococcus* bacterium was not erroneously detected even using the primer set of SEQ ID NOs: 6, 7, 9, 10, and 11 and the probes of SEQ ID NOs: 8 and 12.

### Verification 1: Whole-Genome Analysis Using Next Generation Sequencer and Identification of Bacterial Species Through ANI for Specimens 1 and 2

A DNA-DNA hybridization (DDH) method has long been used to identify bacterial species using gene sequence information, but an average nucleotide identity (ANI) method has currently taken over as a standard technique.

ANI is a technique of identifying bacterial species based on calculation results of determining which reference sequences registered on the database have high sequence similarity with the sequence of the whole genome to be analyzed.

### i) Picking and Genome Extraction From Isolated Colony

A genome was extracted from a supernatant, which was picked up from an isolated colony and treated with phenol-chloroform-isoamyl alcohol (Nippon Genetics Co, Ltd.), using a FastGene Gel/PCR Extraction Kit (Nippon Genetics Co, Ltd.) according to the standard protocol included in the reagent.

### ii) Library Preparation

A library was prepared using a QIAseq FX DNA Library Kit (QIAGEN) according to the standard protocol included in the reagent.

### iii) Sequencing Reaction

A 300bp×2 paired-end sequencing reaction was performed using MiSeq Reagent Kit v3 600 cycles and MiSeq (Illumina, Inc.) as an instrument according to the standard protocol included in the reagent.

### iiii) Identification of Bacterial Species Through ANI

Whole-genome sequencing was performed on raw data acquired from the instrument using Trimmomatic version 0.38 for trimming and SPAdes version 3.13.0 for de novo assembly. The identification of bacterial species was performed through ANI with BBTools using NCBI RefSeq of the National Center for Biotechnology Information as a database.

**The** specimen 1 showed the highest similarity (ANI value: 98.62%) to S. *aureus* on the database, and the next highest was S. *schweitzeri* (ANI value: 93.56%). In addition, the specimen 2 showed the highest similarity (ANI value: 99.09%) to S. *argenteus* on the database, and the next highest was S. *schweitzeri* (ANI value: 94.87%). In general, the threshold value for determining the same bacterial species in the identification of bacterial species through ANI is 95 to 96% (Kim, M, HS Oh, SC Park, J Chun. 2014. Towards a taxonomic coherence between average nucleotide identity and 16S rRNA gene sequence similarity for species demarcation of prokaryotes. Int J Syst Evol Microbiol 64: 346-351.), and with this threshold value, the specimen 1 was identified as S. *aureus* and the specimen 2 was identified as S. *argenteus.* From the above, the validity of the results of Example 2 was supported also from the whole-genome analysis and the identification of bacterial species through ANI.

### Comparative Example 1: Comparison With Prior Art and Investigation of Amplification Efficiency of Primer Sets

S. *argenteus* primers SEQ ID NOs: 13 and 14 (Table 2) disclosed in Patent Publication JP-A-2017-163969 (mentioned above) and SEQ ID NOs: 6, 7, 9, 10, and 11 of the present application were compared in terms of the amplification ability as primer performance.

**[Table 2]**

| | |
|---|---|
| SEQ ID NO: 13: *S*. *argenteus* primer | GAAACAAAGCATCCTAAAAAAGG |
| SEQ ID NO: 14: *S*. *argenteus* primer | AAGCCTTGACGAACTAAAGC |

### Confirmation of Nucleic Acid Amplification

Genomic DNAs extracted from an S. *aureus* JCM5676 strain and an S. *argenteus* JCM31982 strain were used as templates, and amplification by real-time PCR was performed using a primer set of SEQ ID NOs: 13 and 14 or SEQ ID NOs: 6, 7, 9, 10, and 11. TaKaRa Multiplex Assay Kit Ver. 2 (Takara Bio Inc.) was used as a reagent for amplification, and EvaGreen (Biotium) was used as an intercalator dye. The primers and the genomic DNAs were added so that each primer has a final concentration of 0.2 µM and the genomic DNAs as templates have a concentration of 10 µg/L, a 2× reaction buffer was used so as to become a final concentration of 1× in TaKaRa Multiplex Assay Kit Ver. 2, 1 U of Multiplex Enzyme Mix was used, and the template DNAs and the like were mixed with each other to prepare a PCR reaction solution. The measurement was made in triplicate using Mic Real-time PCR (Bio Molecular Systems) as a measurement device.

The conditions used are as follows.
Amplification reaction
94°C: 30 seconds
60°C: 60 seconds -> 94°C: 30 seconds (40 cycles)
72°C: 600 seconds

Thermal melting reaction
95°C: 5 seconds
65°C: 30 seconds
65°C -> 95°C, 0.1°C/second

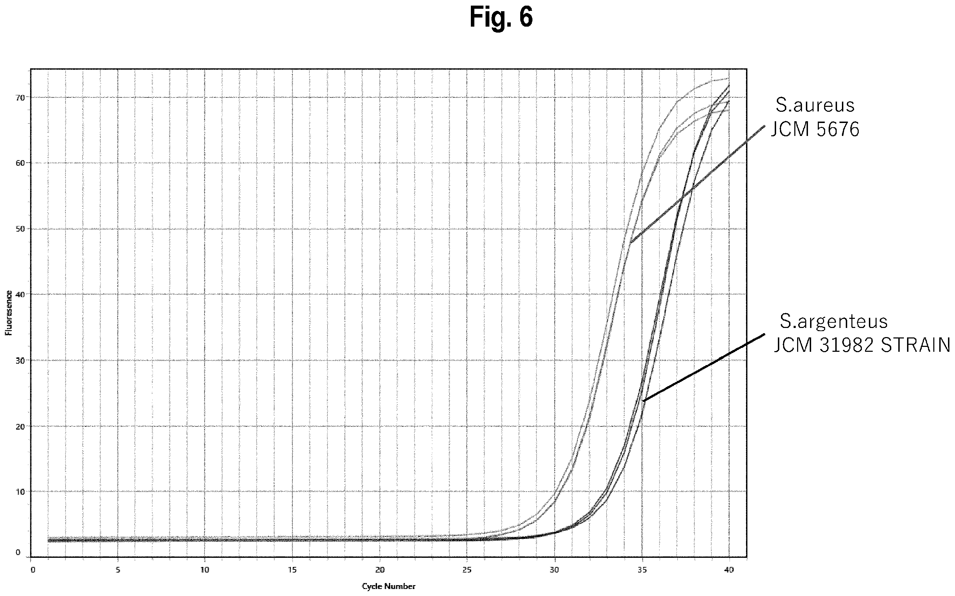

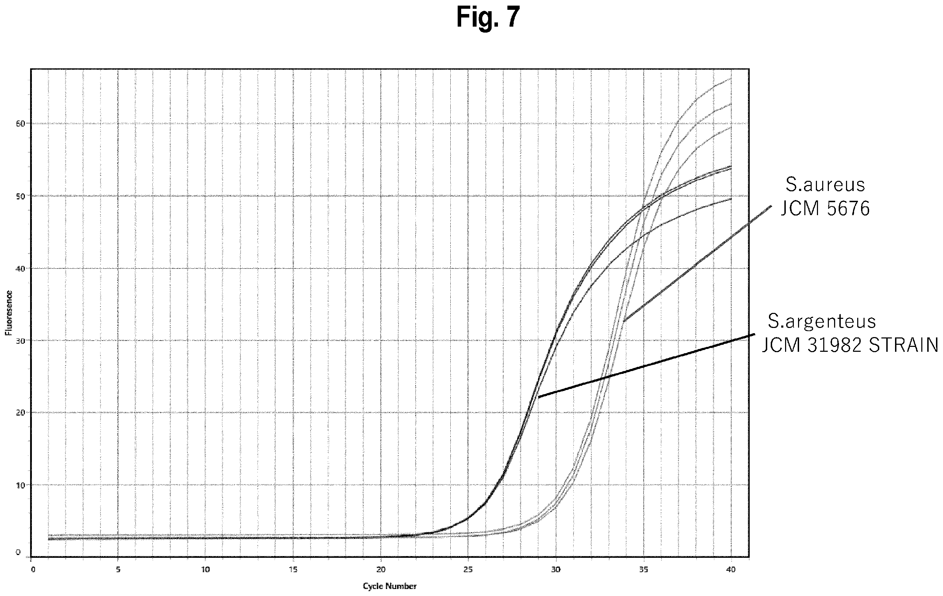

The measurement results when the primer set of SEQ ID NOs: 6, 7, 9, 10, and 11 is used are shown in Fig. 6, and the measurement results when the primer set of SEQ ID NOs: 13 and 14 is used are shown in Fig. 7. Amplification of the S. *aureus* JCM5676 strain and the S. *argenteus* JCM31982 strain was confirmed in both the primer set of SEQ ID NOs: 6, 7, 9, 10, and 11 and the primer set of SEQ ID NOs: 13 and 14. From the above, it was found that the primer set of SEQ ID NOs: 6, 7, 9, 10, and 11 and the primer set of SEQ ID NOs: 13 and 14 have a function of amplifying mutually targeted nucleic acid components.

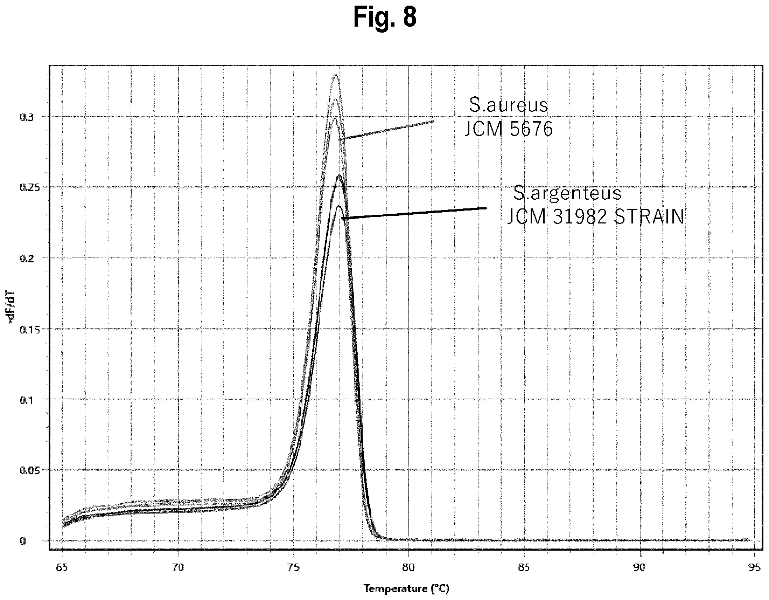

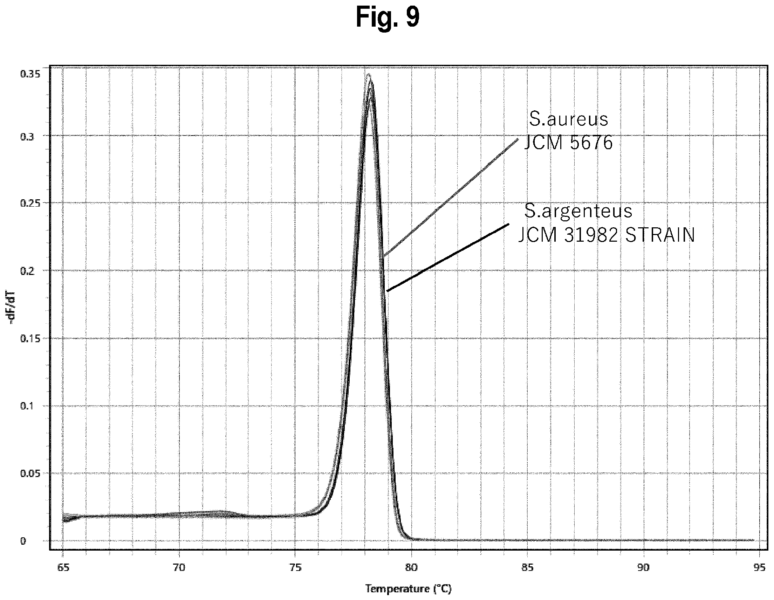

The thermal melting curve analysis results of PCR products when the primer set of SEQ ID NOs: 6, 7, 9, 10, and 11 is used are shown in Fig. 8, and the analysis results when the primer set of SEQ ID NOs: 13 and 14 is used are shown in Fig. 9.

The thermal melting curve analysis is a technique of analyzing components of nucleic acid molecules present in a reaction solution such that the reaction solution is cooled to a low temperature after the PCR reaction, an intercalator is incorporated by forming complementary strands in the solution, and then the reaction solution is heated and the release process of the intercalator due to dissociation of complementary strands is observed by measuring the fluorescence over time. The temperature at which the complementary strands dissociate is a parameter unique to each nucleic acid molecule determined by a base length, a composition, or the like. Therefore, it is possible to confirm what kinds of nucleic acid molecules are present in the solution by checking the temperature, and this parameter is widely used for analyzing nonspecific amplification products shown in primer dimers.

The thermal melting curve analysis results of the PCR products of the S. *aureus* JCM5676 strain and the S. *argenteus* JCM31982 strain amplified by the primer set of SEQ ID NOs: 6, 7, 9, 10, and 11 and SEQ ID NOs: 13 and 14 all showed a unimodal distribution. Since the distribution was unimodal, it was confirmed that the nucleic acid component in the reaction solution was a single component and a target specific amplification product was produced. Moreover, it was found that both primer sets are specific primer sets.

Table 3 shows results obtained by calculating Cq values for comparison in the amplification ability of the primer sets. The Cq values are the number of PCR cycles when the determination threshold value is exceeded. It can be stated that, in a case where the same target is amplified, a primer set with a smaller Cq value has a higher amplification efficiency. It was found that, the Cq value of the S. *argenteus* JCM31982 strain in the primer set of SEQ ID NOs: 6, 7, 9, 10, and 11 was 6.6 lower than that of the primer set of SEQ ID NOs: 13 and 14. From this, it was found that the primer set of SEQ ID NOs: 6, 7, 9, 10, and 11 is superior to the primer set of SEQ ID NOs: 13 and 14 in the amplification of S. *argenteus.* On the other hand, the Cq values of the S. *aureus* JCM5676 strain showed almost no difference between the primer sets, and it was found that both primer sets have the same performance in the amplification of S. *aureus.*

**[Table 3]**

| | *S*. *aureus* (JCM31982) | *S*. *argenteus* (JCM5676) |
|---|---|---|
| SEQ ID NOs: | 27.46±0.15 | 23.30±0.04 |
| 6, 7, 9, 10, and 11 | | |
| SEQ ID NOs: 13 and 14 | 27.20±0.08 | 29.97±0.23 |

### Comparative Example 2: Comparison With Prior Art and Comparison With Differentiating Ability

### Nucleic Acid Amplification and Detection

Genomic DNAs extracted from an S. *aureus* JCM5676 strain and an S. *argenteus* JCM31982 strain were used as templates, and amplification by PCR using a primer set of SEQ ID NOs: 6, 7, 9, 10, and 11 or SEQ ID NOs: 13 and 14, and a hybridization reaction, labeling, and fluorescence measurement on substrates were performed. At this time, primers in which the 5' ends of SEQ ID NOs: 7 and 11 were modified with biotin via linkers were used. The primers and the genomic DNAs were added so that each primer has a final concentration of 0.2 µM and the genomic DNAs as templates have a concentration of 10 µg/L, a 2× reaction buffer was used so as to become a final concentration of 1× in TaKaRa Multiplex Assay Kit Ver. 2, 1 U of Multiplex Enzyme Mix was used, and the template DNAs and the like were mixed with each other to prepare a PCR reaction solution. T100 Thermal Cycler (Bio-Rad Laboratories, Inc.) was used as a nucleic acid amplifier. After the completion of the amplification reaction, a thermal denaturation reaction was performed in the same device before the reactants were subjected to a hybridization reaction on substrates.

As a reagent used for the hybridization reaction on substrates, a solution comprising a substrate 1 on which a probe of SEQ ID NO: 8 was immobilized, a solution comprising a substrate 2 on which a probe of SEQ ID NO: 12 was immobilized, a solution comprising a substrate 3 on which a probe of SEQ ID NO: 15 (ATAAGCTAAGCCACGTCC)was immobilized, Saline-Sodium Phosphate-EDTA Buffer (SSPE buffer), and the PCR reaction solution which had been subjected to thermal denaturation were mixed with each other.

Streptavidin-Phycoerythrin (PlexBio Co., Ltd.) was used as a reagent for labeling. IntelliPlex 1000 πCode Processor (PlexBio Co., Ltd.) was used as a device for the labeling and the hybridization reaction on substrates.

PlexBio (registered trademark) 100 Fluorescent Analayzer (PlexBio Co., Ltd.) was used as a fluorescence measurement device.

The reaction conditions used are as follows.
Amplification reaction conditions
94°C: 30 seconds
60°C: 60 seconds -> 94°C: 30 seconds (30 cycles)
72°C: 600 seconds

Thermal denaturation conditions
95°C: 5 minutes -> rapid cooling to 4°C

Hybridization conditions
37°C: incubation for 20 minutes

Labeling conditions
37°C: incubation for 10 minutes

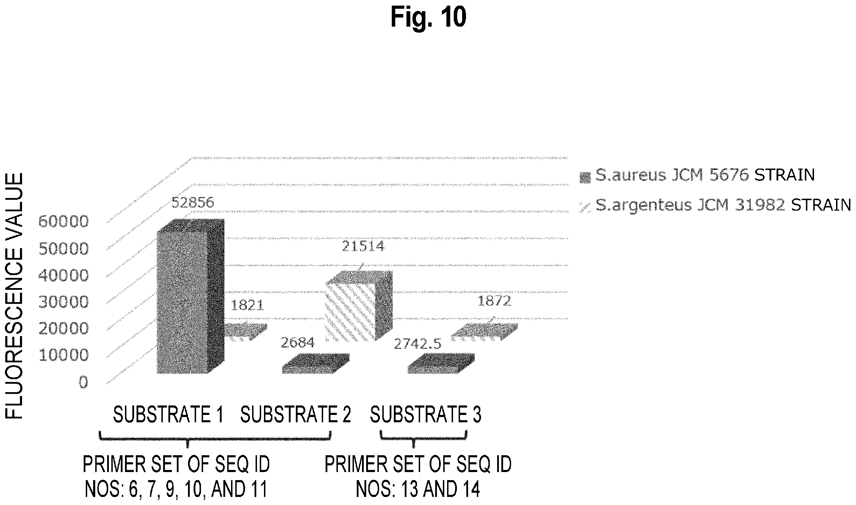

The measurement results are shown in Fig. 10. It is known that the substrate 1 and the substrate 2 have an ability to differentiate S. *aureus* from S. *argenteus* as evaluated in Example 1. Almost no fluorescence is observed in the substrate 3 for both S. *aureus* and S. *argenteus,* and significant detection is almost impossible.

From the above, it was found that the assay of using the primer set of SEQ ID NOs: 6, 7, 9, 10, and 11 in the solution comprising the substrate 1 on which the probe of SEQ ID NO: 8 is immobilized or the substrate 2 on which the probe of SEQ ID NO: 12 is immobilized had an amplification efficiency superior to that of the prior art.

## Claims

1. A primer set for detecting the presence of *Staphylococcus argenteus* in a specimen, the primer set comprising a first primer and a second primer that target a nuc gene of *Staphylococcus argenteus.*

2. The primer set according to claim 1,
wherein the nuc gene has a base sequence shown in SEQ ID NO: 3 or 4 or a base sequence in which one or several bases are deleted, substituted, inserted, or added in the base sequence shown in any of SEQ ID NOs: 3 and 4, and encodes TNase.

3. The primer set according to claim 1 or 2,
wherein the target nuc gene has a base sequence comprising at least 10 consecutive bases in a base sequence from position 217 to position 331 of the base sequence shown in SEQ ID NO: 3 or 4.

4. The primer set according to any one of claims 1 to 3,
wherein the first primer has a base sequence shown in SEQ ID NO: 9 or a base sequence comprising at least 10 consecutive bases in a base sequence in which one or several bases are deleted, substituted, inserted, or added in the base sequence shown in SEQ ID NO: 9, and has the same function as the base sequence shown in SEQ ID NO: 9.

5. The primer set according to any one of claims 1 to 4,
wherein the second primer has a base sequence shown in SEQ ID NO: 10 or a base sequence comprising at least 10 consecutive bases in a base sequence in which one or several bases are deleted, substituted, inserted, or added in the base sequence shown in SEQ ID NO: 10, and has the same function as the base sequence shown in SEQ ID NO: 10.

6. The primer set according to any one of claims 1 to 5, further comprising:
a third primer that targets a nuc gene of *Staphylococcus schweitzeri.*

7. The primer set according to any one of claims 1 to 6,
wherein the third primer has a base sequence shown in SEQ ID NO: 11 or a base sequence comprising at least 10 consecutive bases in a base sequence in which one or several bases are deleted, substituted, inserted, or added in the base sequence shown in SEQ ID NO: 11, and has the same function as the base sequence shown in SEQ ID NO: 11.

8. The primer set according to any one of claims 1 to 7, further comprising:
a fourth primer and a fifth primer that target a nuc gene of *Staphylococcus aureus.*

9. The primer set according to any one of claims 1 to 8,
wherein the fourth primer has a base sequence shown in SEQ ID NO: 6 or a base sequence comprising at least 10 consecutive bases in a base sequence in which one or several bases are deleted, substituted, inserted, or added in the base sequence shown in SEQ ID NO: 6, and has the same function as the base sequence shown in SEQ ID NO: 6.

10. The primer set according to any one of claims 1 to 9,
wherein the fifth primer has a base sequence shown in SEQ ID NO: 7 or a base sequence comprising at least 10 consecutive bases in a base sequence in which one or several bases are deleted, substituted, inserted, or added in the base sequence shown in SEQ ID NO: 7, and has the same function as the base sequence shown in SEQ ID NO: 7.

11. The primer set according to any one of claims 1 to 10,
wherein the primers are labeled with biotin.

12. A probe for detecting the presence of *Staphylococcus argenteus* in a specimen,
wherein the probe targets a nuc gene of *Staphylococcus argenteus.*

13. The probe according to claim 12,
wherein the nuc gene has a base sequence shown in SEQ ID NO: 3 or 4 or a base sequence in which one or several bases are deleted, substituted, inserted, or added in the base sequence shown in SEQ ID NO: 3 or 4, and encodes Nuc protein.

14. The probe according to claim 12 or 13,
wherein the target nuc gene has a base sequence comprising at least 8 consecutive bases in a base sequence from position 217 to position 331 of the base sequence shown in SEQ ID NO: 3 or 4.

15. The probe according to any one of claims 12 to 14,
wherein the probe has a base sequence shown in SEQ ID NO: 12 or a base sequence comprising at least 10 consecutive bases in a base sequence in which one or several bases are deleted, substituted, inserted, or added in the base sequence shown in SEQ ID NO: 12, and has the same function as the base sequence shown in SEQ ID NO: 12.

16. A kit comprising the primer set according to any one of claims 1 to 11, and the probe according to any one of claims 12 to 15.

17. The kit according to claim 16, further comprising:
a probe targeting the nuc gene of *Staphylococcus aureus.*

18. The kit according to claim 17,
wherein the probe targeting the nuc gene of *Staphylococcus aureus* has a base sequence shown in SEQ ID NO: 8 or a base sequence comprising at least 8 consecutive bases in a base sequence in which one or several bases are deleted, substituted, inserted, or added in the base sequence shown in SEQ ID NO: 8, and has the same function as the base sequence shown in SEQ ID NO: 8.

19. A method for detecting the presence of *Staphylococcus argenteus* in a specimen, the method comprising a step of:
using the primer set according to any one of claims 1 to 11.

20. The method according to claim 19, further comprising a step of:
differentiating *Staphylococcus argenteus* from *Staphylococcus schweitzeri* or other *Staphylococcus* bacteria using the first, second, and third primers.

21. The method according to claim 19 or 20,
wherein the probe according to any one of claims 12 to 15 is additionally used.

22. The method according to any one of claims 19 to 21,
wherein the other *Staphylococcus* bacteria include *Staphylococcus aureus* or coagulase-negative *Staphylococcus* bacteria.

23. The method according to any one of claims 19 to 22,
wherein the coagulase-negative *Staphylococcus* bacteria are one or more selected from the group consisting of *Staphylococcus epidermidis, Staphylococcus auricularis, Staphylococcus camosus, Staphylococcus condiment!, Staphylococcus debuckii, Staphylococcus massiliensis, Staphylococcus piscifermentans, Staphylococcus simulans, Staphylococcus capitis, Staphylococcus caprae, Staphylococcus saccharolyticus, Staphylococcus haemolyticus, Staphylococcus devriesei, Staphylococcus hominis, Staphylococcus hyicus, Staphylococcus agnetis, Staphylococcus chromogenes, Staphylococcus cornubiensis*, *Staphylococcus felis, Staphylococcus delphini, Staphylococcus intermedius, Staphylococcus lutrae, Staphylococcus microti, Staphylococcus muscae, Staphylococcus pseudintermedius, Staphylococcus rostri, Staphylococcus schleiferi, Staphylococcus lugudnensis, Staphylococcus saprophyticus, Staphylococcus arlettae, Staphylococcus caeli, Staphylococcus cohnii, Staphylococcus equorum, Staphylococcus gallinarum, Staphylococcus kloosii, Staphylococcus leei, Staphylococcus nepalensis, Staphylococcus succinus, Staphylococcus xylosus, Staphylococcus sciuri, Staphylococcus fleurettii, Staphylococcus lentus, Staphylococcus stepanovicii, Staphylococcus vitulinus, Staphylococcus simulans, Staphylococcus pasteuri,* and *Staphylococcus warneri.*

24. The method according to claim 23,
wherein the other *Staphylococcus* bacteria include *Staphylococcus aureus,* and the fourth primer and the fifth primer and/or the probe that target the nuc gene of *Staphylococcus aureus* are additionally used.
